# EUROPEAN PATENT SPECIFICATION

(11) **EP 4 497 505 B1**
(45) Date of publication and mention of the grant of the patent: **06.05.2026**
(21) Application number: 23211138.5
(22) Date of filing: 21.11.2023
(51) Int. Cl.: B01L 3/02, B01L 3/00

(54) **BIOPARTICLE ENRICHMENT APPARATUS**
GERÄT ZUR ANREICHERUNG VON BIOPARTIKELN
APPAREIL D'ENRICHISSEMENT DE BIOPARTICULES

(30) Priority: 27.07.2023 TW 112128063
(43) Date of publication of application: 29.01.2025
(73) Proprietor: Cytoaurora Biotechnologies, Inc., Zhubei City, Hsinchu County (TW)
(72) Inventor: HUANG, Chung-Er, ZHUBEI CITY, HSINCHU COUNTY (TW); HO, Hsin-Cheng, ZHUBEI CITY, HSINCHU COUNTY (TW)
(74) Representative: Viering, Jentschura & Partner mbB Patent- und Rechtsanwälte

(56) References cited:
- US-A1- 2008 286 751
- US-A1- 2013 319 861
- US-B2- 7 612 355

## Description

### FIELD OF THE INVENTION

The present invention relates to a droplet generator, and more particularly to a bioparticle enrichment apparatus.

### BACKGROUND OF THE INVENTION

An enrichment process is the first step in conventional research for biological particles, but the conventional enrichment process still requires a significant amount of manpower. Accordingly, how apparatuses or devices can be incorporated in the process to quickly and accurately implement the enrichment process has been one of the important areas of research and development in the relevant field.

US 2013/319861A discloses a micro-fluidic device, which can include a processing mechanism for processing micro-objects in a liquid medium and an outputting mechanism for expressing from the device a droplet of the medium containing one or more of the micro-objects. The outputting mechanism can include an expressing mechanism having a reservoir for holding a quantity of the liquid medium and a striking mechanism for striking and compressing the expressing mechanism to express a droplet of the medium from the expressing mechanism.

US 2008/286751A discloses a dispensing device for droplets comprising a first channel (8, 10), known as main channel, for circulation of a first fluid flow, a second channel (12, 13) for circulation of fluid, which forms with the first channel an intersection zone (27) and terminates in an ejection orifice (20), means (4) for measuring a physical property particles or cells in the first channel (18), and means for engendering a pressure wave in the second channel (12, 13).

### SUMMARY OF THE INVENTION

In response to the above-referenced technical inadequacies, the present invention provides a bioparticle enrichment apparatus for effectively improving on the issues associated with conventional enrichment processes according to independent claim 1. The dependent claims show further embodiments of claim 1.

In order to solve the above-mentioned problems, one of the technical aspects adopted by the present invention is to provide a bioparticle enrichment apparatus provided for selecting at least one target bioparticle from a liquid specimen having the bioparticles. The bioparticle enrichment apparatus includes a pico-droplet generator, a power device, a pressure balance device, a camera device, a control device, and a light source mechanism. The pico-droplet generator includes a light sensing structure, a mating structure, a bonding layer, and a piezoelectric member. The light sensing structure includes a first substrate, a first electrode layer and a photoelectric layer respectively formed on two opposite sides of the first substrate, and an insulating layer covering the photoelectric layer. The mating structure is spaced apart from the light sensing structure. At least one of the mating structure and the light sensing structure is transparent, and the mating structure includes a second substrate and a second electrode layer that is formed on the second substrate. The second electrode layer faces toward the insulating layer. The bonding layer is connected in-between the light sensing structure and the mating structure along a thickness direction so as to jointly define a selection channel along a flowing direction perpendicular to the thickness direction. At least one of the mating structure and the bonding layer has an inlet that is located at an upstream of the selection channel and an outlet that is located at a downstream of the selection channel. The bonding layer has a selection hole that is in spatial communication with the selection channel along a dripping direction perpendicular to the thickness direction and the flowing direction. The piezoelectric member is disposed on the bonding layer. The piezoelectric member and the selection hole are respectively located at two opposite sides of the bonding layer along the dripping direction. The pico-droplet generator has a pico-droplet emission region defined as extending from the selection hole toward the piezoelectric member. The power device is electrically coupled to the first electrode layer and the second electrode layer. The pressure balance device includes a first valve that is assembled to the inlet and a second valve that is assembled to the outlet. The pressure balance device is configured to control a velocity and a pressure of the liquid specimen in the selection channel through the first valve and the second valve. The camera device corresponds in position to a viewable segment of the selection channel. The pico-droplet emission region is arranged in the viewable segment, and the camera device is configured to take a real-time image of the liquid specimen in the viewable segment. The control device is electrically coupled to the piezoelectric member. When the real-time image obtained by the camera device shows that at least one target bioparticle is located in the pico-droplet emission region, the control device allows the piezoelectric member to output a pico-droplet by driving the liquid specimen in the pico-droplet emission region to pass through the selection hole, and the pico-droplet covers the at least one target bioparticle. The light source mechanism is in cooperation with the pico-droplet generator. The photoelectric layer includes a collector layer, a plurality of base regions, and a plurality of emitter regions. The collector layer is formed on the first substrate. The collector layer includes a plurality of collector regions spaced apart from each other, and an end of each of the collector regions away from the first electrode layer has a first slot-like portion. The base regions are respectively formed in the first slot-like portions of the collector regions. An end of each of the base regions away from the first electrode layer has a plurality of second slot-like portions spaced apart from each other. The emitter regions are respectively formed in the base regions. Each of the emitter regions includes a plurality of emitter pads respectively formed in the second slot-like portions of a corresponding one of the base regions. Each of the base regions, a corresponding one of the collector regions, and a corresponding one of the emitter regions are jointly formed as a vertical transistor. The insulating layer covers the vertical transistors and separates the vertical transistors from each other, and an end of each of the emitter pads away from the first electrode layer is exposed from the insulating layer. Any one of the vertical transistors is configured to be irradiated by the light source mechanism so as to enable a plurality of DEP forces to be applied to move the at least one target bioparticle through a distribution of the emitter pads and an electric field difference that is generated from non-uniform electric fields of the emitter pads.

Therefore, the configuration of the pico-droplet generator of the bioparticle enrichment apparatus provided by the present invention can be used to quickly and accurately implement a selection and enrichment process of the bioparticle (or the at least one target bioparticle) through the piezoelectric member. The pico-droplet generator in the present invention has an architecture that allows for mass-production of the same, thereby facilitating reduction of the overall cost of the bioparticle enrichment apparatus.

Moreover, the pico-droplet generator and the light source mechanism of the bioparticle enrichment apparatus provided by the present invention can be used in cooperation with each other to allow the pico-droplet emission region to quickly and accurately have the at least one target bioparticle therein, such that the at least one target bioparticle can be collected in the first container by forming the pico-droplet.

These and other aspects of the present invention will become apparent from the following description of the embodiment taken in conjunction with the following drawings and their captions, although variations and modifications therein may be affected without departing from the scope of the novel concepts of the invention.

### BRIEF DESCRIPTION OF THE DRAWINGS

The described embodiments may be better understood by reference to the following description and the accompanying drawings, in which:
FIG. 1 is a schematic planar view of a bioparticle enrichment apparatus according to a first non-claimed example;
FIG. 2 is a schematic perspective view showing a part of FIG. 1;
FIG. 3 is a schematic cross-sectional view taken along line III-III of FIG. 2;
FIG. 4 is a schematic cross-sectional view showing a second valve of FIG. 3 being switched to a closed mode;
FIG. 5 is a schematic cross-sectional view showing a dielectrophoretic force in FIG. 4 applied to move bioparticles;
FIG. 6 is a schematic cross-sectional view showing a pico-droplet generator of FIG. 5 that outputs a pico-droplet through a piezoelectric member;
FIG. 7 is a schematic cross-sectional view showing the second valve of FIG. 6 being switched to an open mode;
FIG. 8 is a schematic cross-sectional view showing the bioparticle enrichment apparatus in another operation process according to the first embodiment of the present invention;
FIG. 9 is a schematic cross-sectional view showing the pico-droplet generator of FIG. 8 that outputs a pico-droplet through the piezoelectric member;
FIG. 10 is a schematic perspective view showing a part of the bioparticle enrichment apparatus according to a first claimed embodiment of the present invention;
FIG. 11 is a schematic cross-sectional view taken along line XI-XI of FIG. 10;
FIG. 12 is a schematic enlarged view of part XII of FIG. 11; and
FIG. 13 is a schematic top view of a vertical transistor of FIG. 12.

### DETAILED DESCRIPTION OF THE EXEMPLARY EMBODIMENTS

The present invention is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a," "an" and "the" includes plural reference, and the meaning of "in" includes "in" and "on." Titles or subtitles can be used herein for the convenience of a reader, which shall have no influence on the scope of the present invention.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present invention or of any exemplified term. Likewise, the present invention is not limited to various embodiments given herein. Numbering terms such as "first," "second" or "third" can be used to describe various components, signals or the like, which are for distinguishing one component/signal from another one only, and are not intended to, nor should be construed to impose any substantive limitations on the components, signals or the like.

### [First Example]

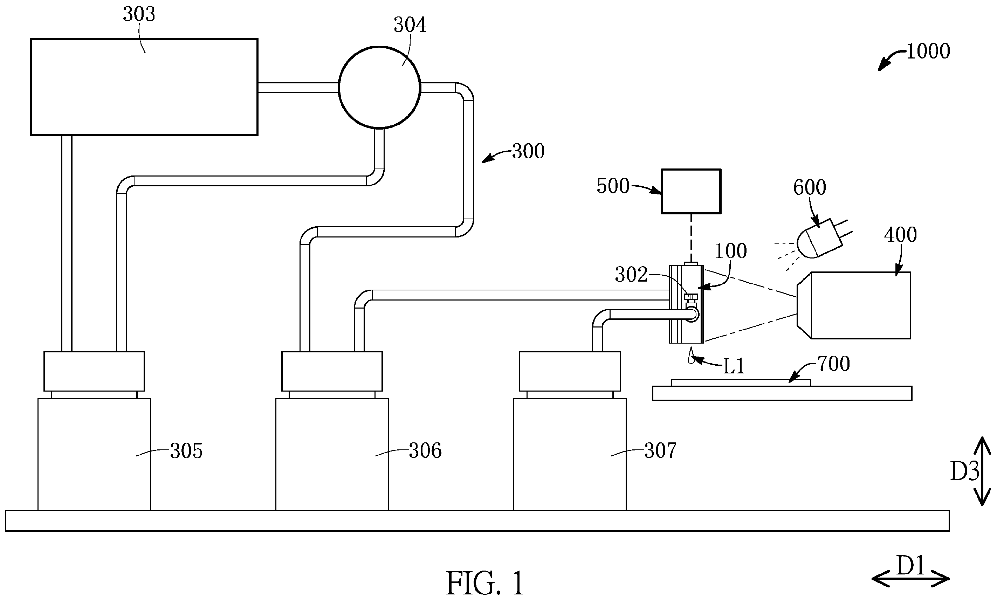

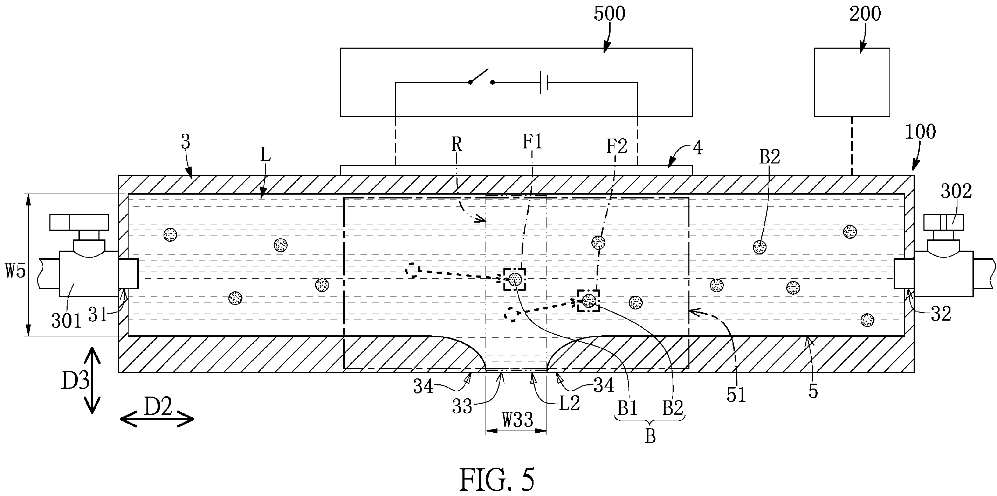

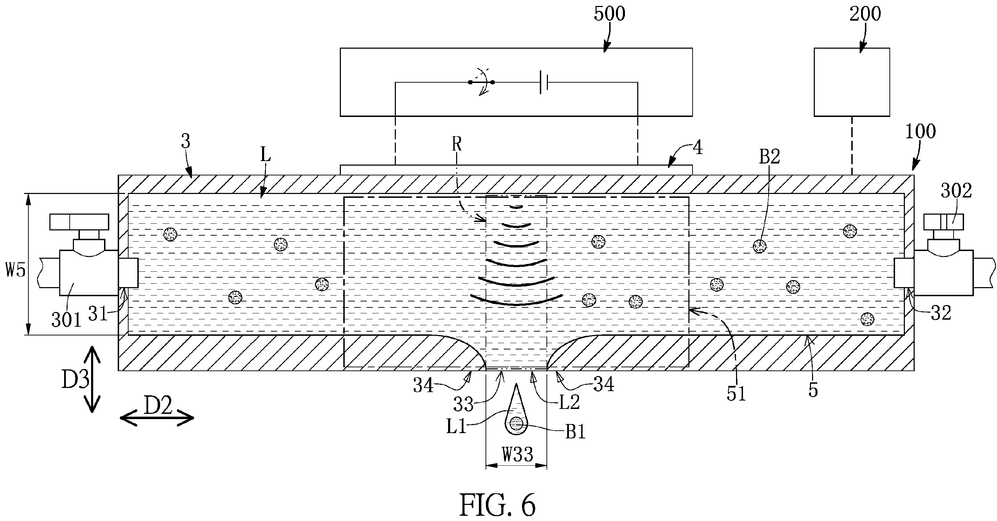

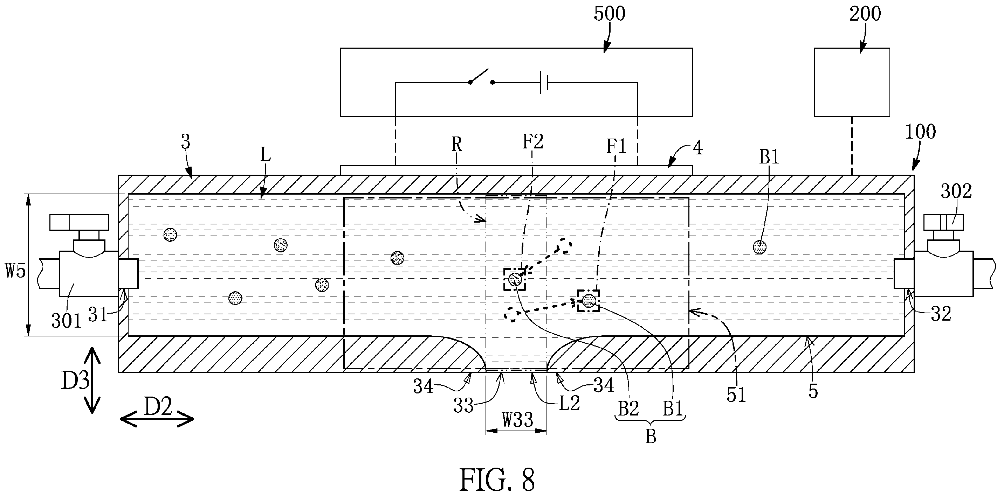

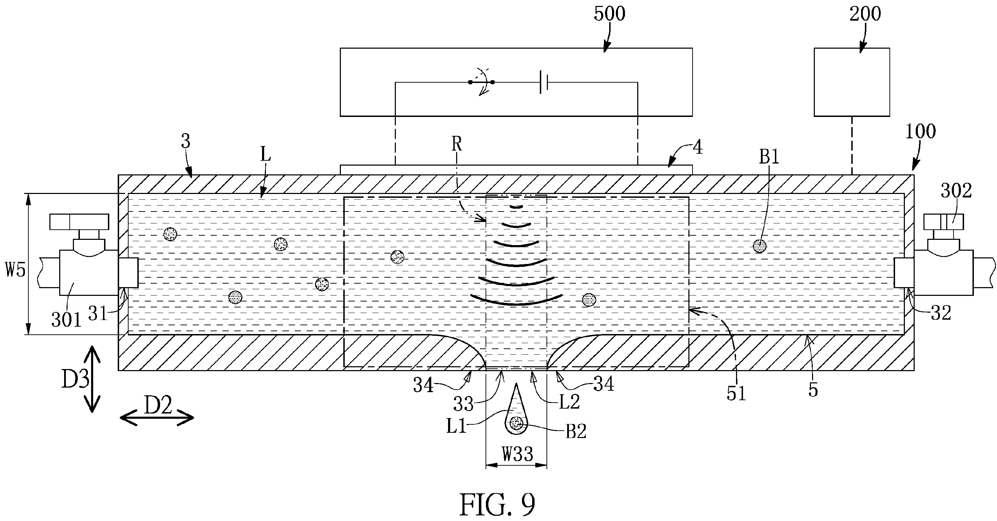

Referring to FIG. 1 to FIG. 9, a first non-claimed example is provided. As shown in FIG. 1 to FIG. 3, the present example provides a bioparticle enrichment apparatus 1000 for selecting at least one of bioparticles B from a liquid specimen L having the bioparticles B, thereby enabling the at least one of the bioparticles B to be collected in a first container

In the present example, the liquid specimen L can be a body fluid from an animal (e.g., blood, lymph, saliva, ascites, or urine), and the bioparticle B can be a specific type of cell, such as circulating tumor cells (CTCs), fetal nucleated red blood cells (FNRBCs), exosomes, virus, or bacteria, but the present invention is not limited thereto. For example, in other examples not shown in the drawings, the liquid specimen L can also be obtained from plants.

Moreover, the bioparticle enrichment apparatus 1000 in the present example includes a pico-droplet generator 100, a power device 200 electrically coupled to the pico-droplet generator 100, a pressure balance device 300 being in spatial communication with an interior space of the pico-droplet generator 100, a camera device 400 provided for observing the pico-droplet generator 100, a control device 500 provided for driving (or triggering) the pico-droplet generator 100, and a light source mechanism 600 that is used in cooperation with the pico-droplet generator 100 in a contactless manner, but the present invention is not limited thereto.

For example, in other examples not shown in the drawings, configurations and number of inner components of the bioparticle enrichment apparatus 1000 can be changed or adjusted according to design requirements (e.g., the light source mechanism 600 can be omitted), and the pico-droplet generator 100 can be independently used (e.g., sold) or can be used in cooperation with other devices (e.g., the pico-droplet generator 100 and the light source mechanism 600 can be used in cooperation with each other and are jointly defined as a bioparticle enrichment device).

The following description describes the structure and connection relationship of each component of the bioparticle enrichment apparatus 1000, and then describes the connection relationship of each component of the bioparticle enrichment apparatus 1000. It should be noted that the pico-droplet generator 100 of the present example is formed at a chip-scale (e.g., a thickness of the pico-droplet generator 100 is within a range from 600 µm to 800 µm), and the pico-droplet generator 100 shown in the drawings is a rectangular structure, but the present disclosure is not limited thereto. For example, in other examples not shown in the drawings, the pico-droplet generator 100 can be a curved structure or an irregular structure.

Specifically, the pico-droplet generator 100 in the present example includes a light sensing structure 1, a mating structure 2 that is spaced apart from the light sensing structure 1, a bonding layer 3 that is connected to and located between the light sensing structure 1 and the mating structure 2 along a thickness direction D1, and a piezoelectric member 4 that is disposed on the bonding layer 3. In order to clearly describe the pico-droplet generator 100, the mating structure 2 in the present example is transparent. According to practical requirements, at least one of the mating structure 2 and the light sensing structure 1 can be transparent so as to enable the pico-droplet generator 100 to be normally operated.

The light sensing structure 1 includes a first substrate 11, a first electrode layer 12 formed on one side (e.g., a bottom side) of the first substrate 11, a photoelectric layer 13 formed on another side (e.g., a top side) of the first substrate 11, and an insulating layer 14 that covers at least part of the photoelectric layer 13. In the present example, the first substrate 11 can be a silicon substrate and is preferably a low-doped N-type layer, the first electrode layer 12 can cover an entirety of the bottom side of the first substrate 11, and the first electrode layer 12 is preferably a thin conductive metal layer or an indium tin oxide (ITO) layer. Moreover, configuration or layout of the photoelectric layer 13 can be adjusted or changed according to design requirements, and the present invention is not limited thereto. For example, the photoelectric layer 13 can have a plurality of vertical transistors (now labeled in the drawings) in a matrix arrangement, and further details of the vertical transistors of the photoelectric layer 13 are described in the following first embodiment for the sake of brevity.

The mating structure 2 includes a second substrate 21 and a second electrode layer 22 that is formed on the second substrate 21 and that faces toward the light sensing structure 1. The bonding layer 3 is connected to and arranged between the light sensing structure 1 and the mating structure 2 so as to jointly define a selection channel 5 along a flowing direction D2 that is perpendicular to the thickness direction D1.

Specifically, at least one of the mating structure 2 and the bonding layer 3 has an inlet 31 and an outlet 32, which are respectively in spatial communication with two ends of the selection channel 5. In other words, the inlet 31 is located at an upstream of the selection channel 5, and the outlet 32 is located at a downstream of the selection channel 5. Moreover, the inlet 31 and the outlet 32 in the present example are formed on the bonding layer 3, and the liquid specimen L can be injected into the pico-droplet generator 100 through the inlet 31 and can flow out of the pico-droplet generator 100 through the outlet 32.

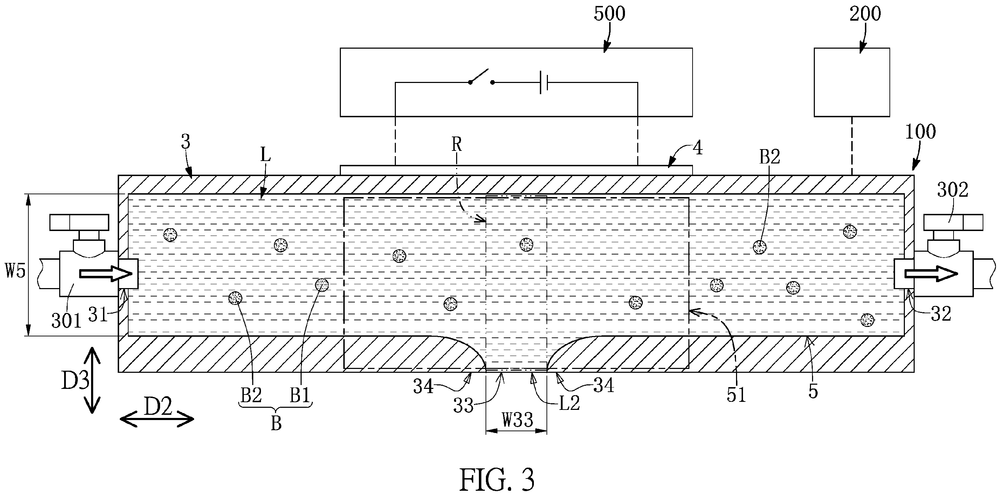

Furthermore, the bonding layer 3 has a selection hole 33 that is in spatial communication with the selection channel 5 along a dripping direction D3 perpendicular to the thickness direction D1 and the flowing direction D2 (e.g., the selection hole 33 penetrates through the bonding layer 3 along the dripping direction D3). In the present example, the pico-droplet generator 100 preferably has a hydrophobic surface 34 surrounding the selection hole 33 (i.e., the selection hole 33 is surrounded and defined by the hydrophobic surface 34). The hydrophobic surface 34 in the present example includes bottom edges of the bonding layer 3 (as shown in FIG. 3) respectively located at two opposite sides of the selection hole 33 along the flowing direction D2, a partial outer surface of the light sensing structure 1, and a partial outer surface of the mating structure 2, the latter two of which are respectively located at two opposite side of the selection hole 3 along the thickness direction D1 (not shown in the drawings). Moreover, the selection hole 33 is substantially arranged on a center portion of the bonding layer 3 along the flowing direction D2, a width W33 of the selection hole 33 along the flowing direction D2 is within a range from 40 µm to 300 µm, and the dripping direction D3 is substantially perpendicular to a horizontal plane or substantially parallel to a vertical line, but the present invention is not limited thereto.

The piezoelectric member 4 is disposed on a top edge of the bonding layer 3 and is located outside of the selection channel 5, and the piezoelectric member 4 and the selection hole 33 are respectively located at two opposite sides of the bonding layer 3 along the dripping direction D3. The pico-droplet generator 100 (or the selection channel 5) has a pico-droplet emission region R defined as extending from the selection hole 33 toward the piezoelectric member 4.

It should be noted that the pico-droplet emission region R in the present example is arranged in a projection area defined by orthogonally projecting the selection hole 33 onto the piezoelectric member 4 along the dripping direction D3, and a boundary of the pico-droplet emission region R is spaced apart from a center of the selection hole 33 along the dripping direction D3 by a distance within a range from 50 µm to 200 µm. In other words, the pico-droplet emission region R can cover an entirety of a width W5 of the selection channel 5 along the dripping direction D3, and a volume of the pico-droplet emission region R can be substantially equal to that of at least one pico-droplet L1 according to design requirements.

In addition, the boundary of the pico-droplet emission region R can be changed or adjusted according to design requirements and is not limited by the drawings. For example, in other examples not shown in the drawings, the pico-droplet emission region R can have the shape of a circular-sector defined as extending from the selection hole 3; or, the pico-droplet emission region R covers a part (e.g., 30% to 80%) of the width W5 of the selection channel 5 along the dripping direction D3.

The power device 200 in the present example is an alternating current (AC) power device. The power device 200 is electrically coupled to the first electrode layer 12 and the second electrode layer 22 of the pico-droplet generator 100, thereby providing electricity for a cooperative operation of the pico-droplet generator 100 and the light source mechanism 600.

The pressure balance device 300 includes a first valve 301 that is assembled to the inlet 31 and a second valve 302 that is assembled to the outlet 32. The pressure balance device 300 is configured to control a velocity and a pressure of the liquid specimen L in the selection channel 5 through the first valve 301 and the second valve 302. For example, the pressure balance device 300 enables the liquid specimen L to form a liquid level L2 in the selection hole 33 that is coplanar with an outer surface (e.g., the bottom edges) of the bonding layer 3.

Specifically, the pressure balance device 300 in the present example includes an air pump 303, a switch 304 connected to the air pump 303, a pressure balance bottle 305 being in fluid communication with the air pump 303 and the switch 304, a liquid injection bottle 306 being in fluid communication with the switch 304 and the first valve 301, and a second container 307 that is in fluid communication with the second valve 302. The liquid injection bottle 306 enables the liquid specimen L received therein to be injected into the selection channel 5 through the first valve 301 and the inlet 31. The second container 307 is provided to receive the liquid specimen L that is selected (or filtered) and flows out of the selection channel 5 from the outlet 32 and the second valve 302.

The camera device 400 corresponds in position to a viewable segment 51 of the selection channel 5, and the camera device 400 is preferably configured to take a real-time image of the liquid specimen L in the viewable segment 51, thereby determining a preferable operation time of the pico-droplet generator 100. It should be noted that the bioparticles B located in the viewable segment 51 can be divided into at least one target bioparticle B1 and at least one non-target bioparticle B2. Moreover, the camera device 400 faces toward the light sensing structure 1 being transparent or the mating structure 2 being transparent along thickness direction D1, thereby clearly observing a real-condition of the liquid specimen L that flows in the selection channel 5 along the flowing direction D2.

Specifically, a boundary of the viewable segment 51 is spaced apart from a center of the selection hole 33 along the flowing direction D2 by a distance within a range from 100 µm to 300 µm, the viewable segment 51 covers an entirety of the width W5 of the selection channel 5 along the dripping direction D3, and the pico-droplet emission region R is arranged in the viewable segment 51. In other words, the viewable segment 51 can be defined as a filtering segment, a portion of the selection channel 5 arranged at an upstream of the viewable segment 51 can be defined as an unfiltered segment, and another portion of the selection channel 5 arranged at a downstream of the viewable segment 51 can be defined as a filtered segment, but the present invention is not limited thereto.

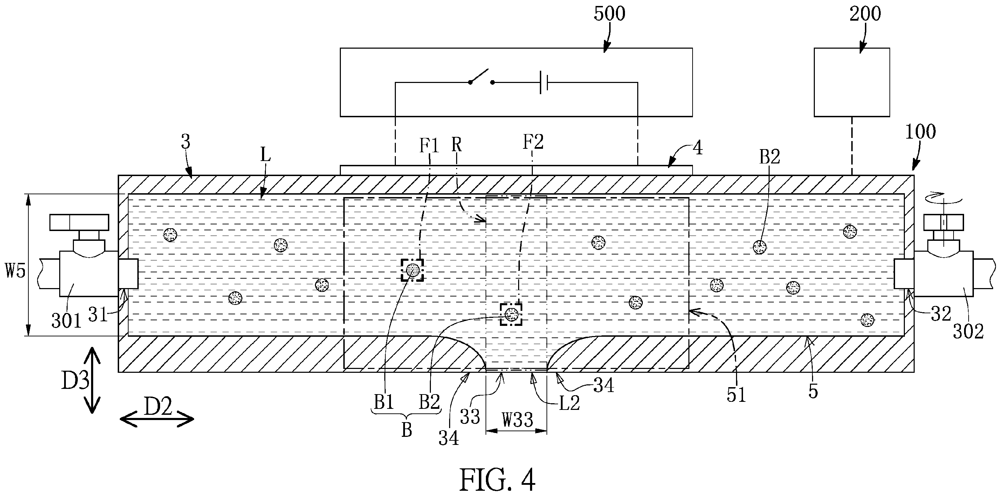

The control device 500 is electrically coupled to the piezoelectric member 4 of the pico-droplet generator 100, so that the control device 500 can be used in cooperation with the real-time image obtained by the camera device 400 to trigger (or drive) the piezoelectric member 4. It should be noted that when the piezoelectric member 4 (or the light source mechanism 600) is in operation, the pressure balance device 300 maintains the pressure of the liquid specimen L in the selection channel 5 through the first valve 301 in an open mode and the second valve 302 in a closed mode (as shown in FIG. 4 to FIG. 6).

As shown in FIG. 1, FIG. 5, and FIG. 6, when the real-time image obtained by the camera device 400 shows that at least one of the bioparticles B (e.g., the at least one target bioparticle B1) is located in the pico-droplet emission region R, the control device 500 allows the piezoelectric member 4 to output one of the pico-droplets L1 by driving the liquid specimen L in the pico-droplet emission region R to pass through the selection hole 33. Moreover, the pico-droplet L1 covers the at least one of the bioparticles B (e.g., the at least one target bioparticle B1) and is received (or collected) in the first container 700.

In addition, if the bioparticle B (e.g., the at least one target bioparticle B1) is located away from the selection hole 33, the control device 500 enables the piezoelectric member 4 to sequentially output more than one of the pico-droplets L1 for ensuring that at least one of the pico-droplets L1 outputted from the pico-droplet generator 100 covers the bioparticle B (e.g., the at least one target bioparticle B1).

Accordingly, the configuration of the pico-droplet generator 100 of the bioparticle enrichment apparatus 1000 provided by the present example can be used to quickly and accurately implement a selection and enrichment process of the bioparticle B (e.g., the at least one target bioparticle B1) through the piezoelectric member 4. The pico-droplet generator 100 in the present example has an architecture that allows for mass-production of the same, thereby facilitating reduction of overall cost of the bioparticle enrichment apparatus 1000.

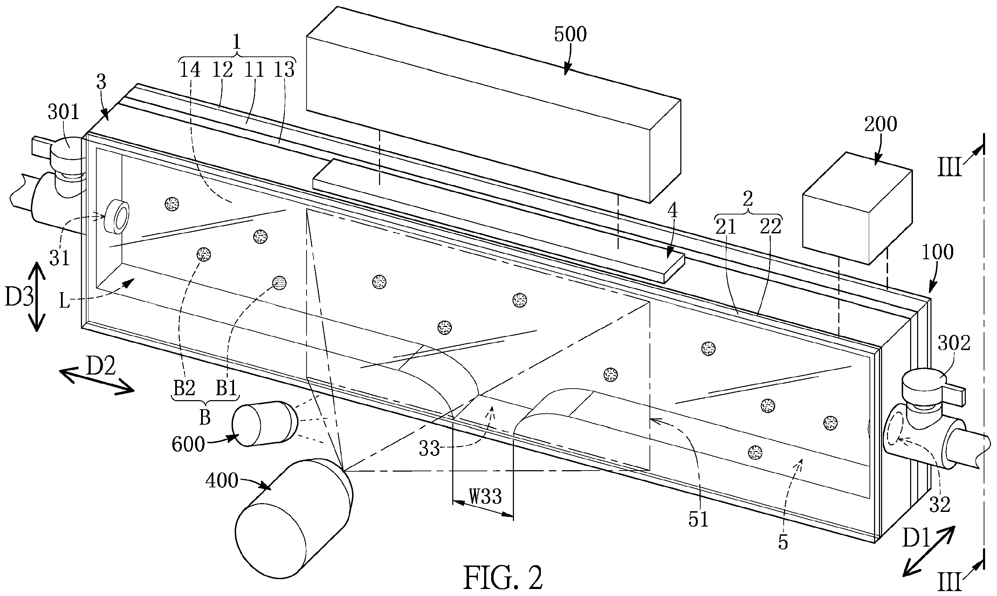

In addition, in order to enable the pico-droplet generator 100 to accurately form the pico-droplet L1 covering the at least one target bioparticle B1 for effectively implement the selection and enrichment process of the at least one target bioparticle B1, the pico-droplet generator 100 is preferably used in cooperation with the light source mechanism 600. As shown in FIG. 2, FIG. 4, and FIG. 5, the light source mechanism 600 is arranged adjacent to the camera device 400, and the light source mechanism 600 is configured to move the at least one target bioparticle B1 into the pico-droplet emission region R by emitting light onto the at least one target bioparticle B1 to apply a dielectrophoretic (DEP) force F1 to the at least one target bioparticle B1 through the light sensing structure 1. Moreover, the light source mechanism 600 can be used to move the at least one non-target bioparticle B2 away from the pico-droplet emission region R by emitting light onto the at least one non-target bioparticle B2 to apply a DEP force F2 to the at least one non-target bioparticle B1 through the light sensing structure 1.

Accordingly, the pico-droplet generator 100 and the light source mechanism 600 of the bioparticle enrichment apparatus 1000 provided by the present example can be used in cooperation with each other to allow the pico-droplet emission region R to quickly and accurately have the at least one target bioparticle B1 therein, such that the at least one target bioparticle B1 can be collected in the first container 700 by forming the pico-droplet L1.

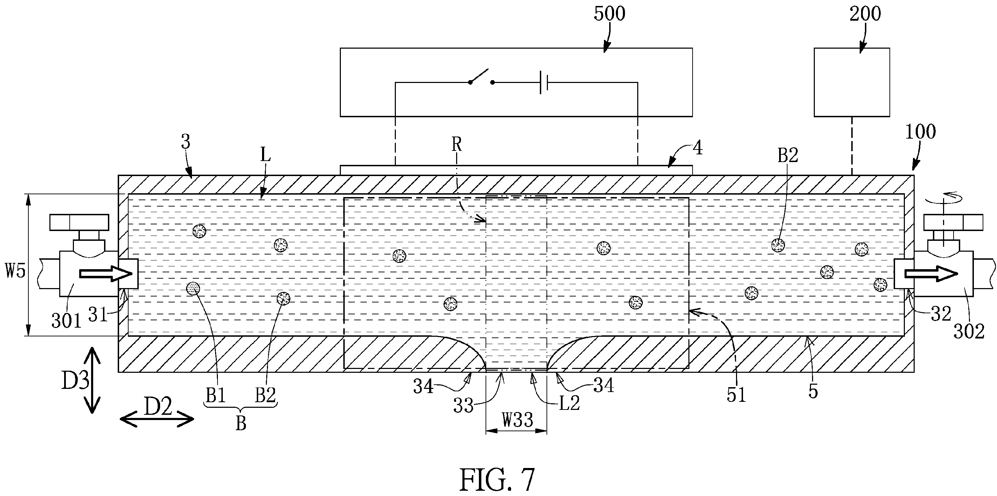

As shown in FIG. 1 and FIG. 7, after the at least one target bioparticle B1 located in the pico-droplet emission region R is collected in the first container 700, the second valve 302 can be opened again for allowing the liquid specimen L in the viewable segment 51 not having any target bioparticle B1 to flow into the second container 307, so that the non-target bioparticles B2 can be collected in the second container 307, but the present invention is not limited thereto.

For example, as shown in FIG. 1, FIG. 8, and FIG. 9, the light source mechanism 600 can be configured to move the at least one target bioparticle B1 away from pico-droplet emission region R by emitting light onto the at least one target bioparticle B1 and can be configured to move the at least one non-target bioparticle B2 into the pico-droplet emission region R by emitting light onto the at least one non-target bioparticle B2, such that the at least one non-target bioparticle B2 is collected in the first container 700 by forming the pico-droplet L1, the at least one target bioparticle B1 is collected in the second container 307.

In addition, a quantity of the selection hole 33 of the pico-droplet generator 100 in the present example is only one, but the present invention is not limited thereto. For example, in other examples not shown in the drawings, the pico-droplet generator 100 can have a plurality of selection holes 33 spaced apart from each other and arranged along the flowing direction D2, and the pico-droplet generator 100 can further include a plurality of operation mechanisms (e.g., the piezoelectric members 4) respectively operated with the selection holes 33, so that the bioparticle enrichment apparatus 1000 can be used to select more than one kind of the bioparticles B according to different requirements through the selection holes 33 and the operation mechanisms.

### [First Embodiment]

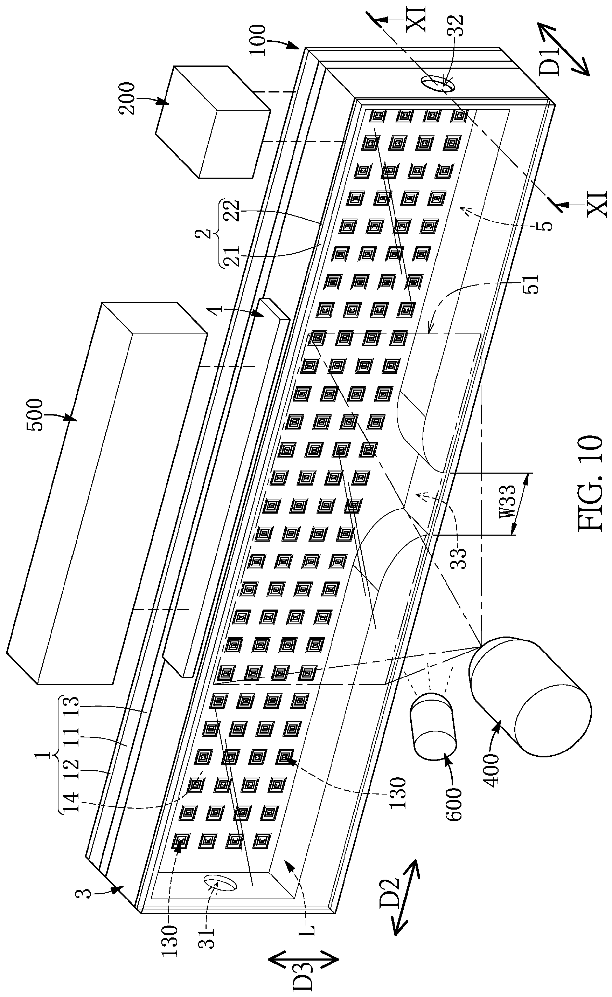

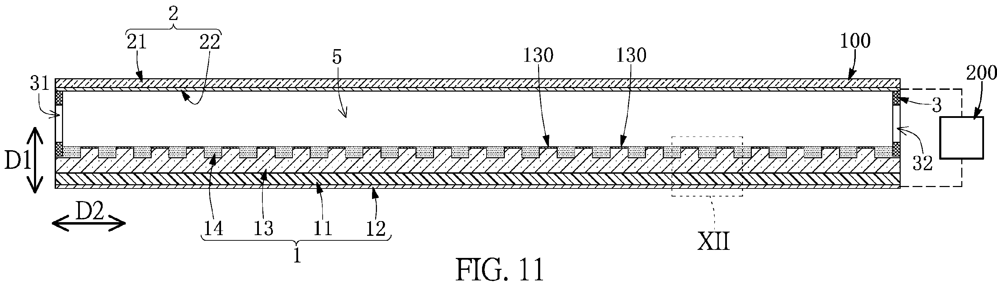

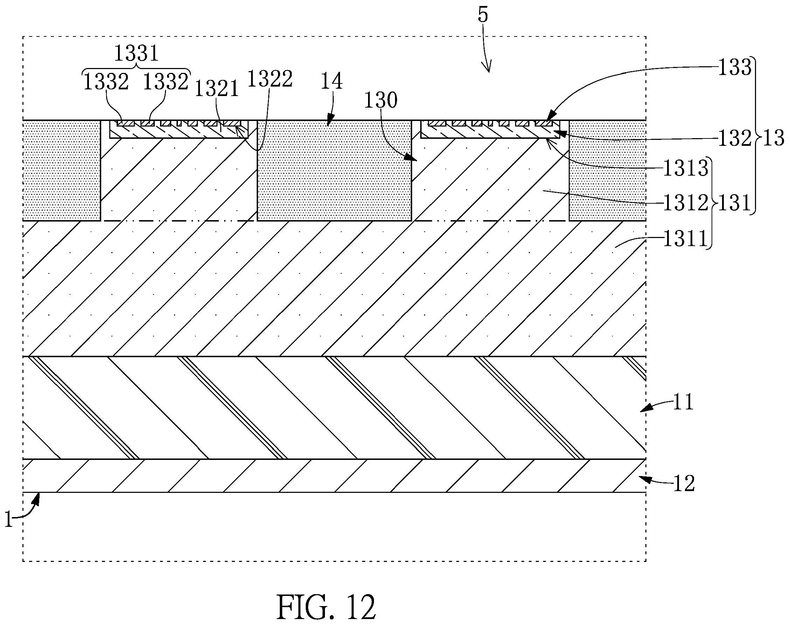

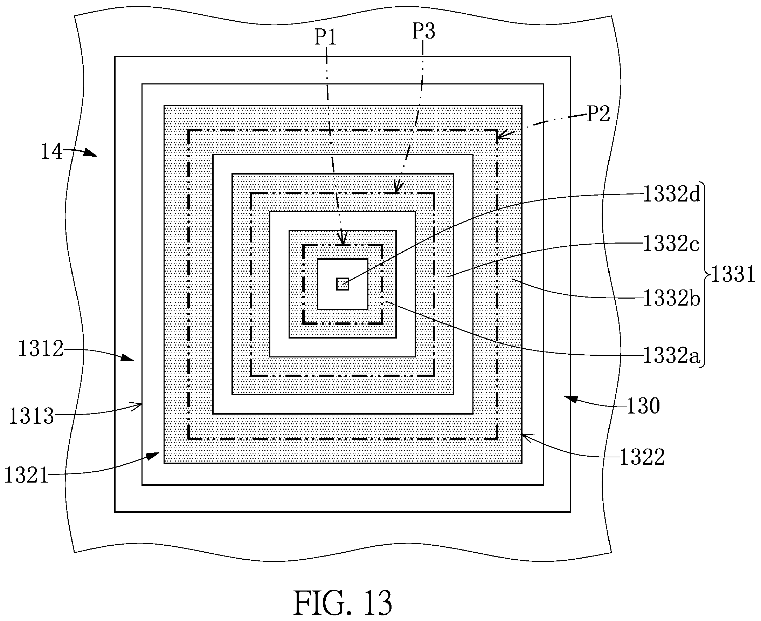

Referring to FIG. 10 to FIG. 13, a first claimed embodiment of the present invention, which is similar to the first example, is provided. For the sake of brevity, descriptions of the same components in the first example and first embodiment of the present invention will be omitted herein, and the following description only discloses different features between the first example and first embodiment.

In the present embodiment, the photoelectric layer 13 can generate a better photoelectric coupling effect through a specific configuration. Specifically, the photoelectric layer 13 includes a collector layer 131 formed on the first substrate 11, a plurality of base regions 1321 formed in the collector layer 131, and a plurality of emitter regions 1331 that are respectively formed in the base regions 1321. In other words, the collector layer 131 is a N-type layer; the base regions 1321 are located at a same height with respect to the first electrode layer 12 and are jointly defined as a base layer 132 that is a P-type layer; and the emitter regions 1331 are located at a same height with respect to the first electrode layer 12 and are jointly defined as an emitter layer 133 that is a heavily doped N-type layer.

Specifically, the collector layer 131 in the present embodiment includes a connection layer 1311 formed on the first substrate 11 and a plurality of collector regions 1312 that are formed on the connection layer 1311 and that are spaced apart from each other. Moreover, an end (e.g., a top end) of each of the collector regions 1312 away from the first electrode layer 12 has a first slot-like portion 1313.

It should be noted that the collector regions 1312 of the present embodiment are electrically coupled to each other through the connection layer 1311, but the present invention is not limited thereto. For example, in other embodiments of the present invention not shown in the drawings, according to design requirements, the collector layer 131 can be provided without the connection layer 1311, the collector regions 1312 are directly formed on the first substrate 11, and the first substrate 11 can be a low-doped N-type layer, so that the first substrate 11 and the collector layer 131 can be jointly used as a collector.

The base regions 1321 are respectively formed in the first slot-like portions 1313 of the collector regions 1312 (i.e., each of the collector regions 1312 is formed with one of the base regions 1321 arranged therein), and an end (e.g., a top end) of each of the base regions 1321 away from the first electrode layer 12 has a plurality of second slot-like portions 1322 spaced apart from each other.

Moreover, the emitter regions 1331 are respectively formed in the base regions 1321 (i.e., each of the base regions 1321 is formed with one of the emitter regions 1331 arranged therein). Each of the emitter regions 1331 includes a plurality of emitter pads 1332 respectively formed in the second slot-like portions 1322 of a corresponding one of the base regions 1321.

In summary, each of the base regions 1321, a corresponding one of the collector regions 1312, and a corresponding one of the emitter regions 1331 are jointly formed as a vertical transistor 130. The insulating layer 14 in the present embodiment is a silicon nitride layer or a silicon oxide layer, but the present invention is not limited thereto. The insulating layer 14 covers the vertical transistors 130 and separates the vertical transistors 130 from each other, and an end (e.g., a top end) of each of the emitter pads 1322 away from the first electrode layer 12 is exposed from the insulating layer 14. In other words, the insulating layer 14 covers and is connected to the connection layer 1311 and a surrounding lateral surface of each of the vertical transistors 130.

As the vertical transistors 130 in the present embodiment are of substantially the same structure, the following description discloses the structure of just one of the vertical transistors 130 for the sake of brevity, but the present invention is not limited thereto. For example, in other embodiments of the present invention not shown in the drawings, the vertical transistors 130 can be of different structures.

In the present embodiment, the end of the collector region 1312, the end of the base region 1321, and the end of each of the emitter pads 1332 are preferably coplanar with each other, each of the emitter pads 1332 has a width greater than a thickness thereof, and any two of the emitter pads 1332 adjacent to each other have a distance therebetween that is less than 5 µm, but the present invention is not limited thereto. In addition, a thickness of the base region 1321 is within a range from 15% to 35% of a thickness of the collector region 1312, and the thickness of each of the emitter pads 1332 is within a range from 5% to 20% of the thickness of the base region 1321, but the present invention is not limited thereto.

It should be noted that from the perspective of the bioparticles, any slight change in the pico-droplet generator 100 would have a significant influence thereon. Accordingly, the above description in the present embodiment describes the size and arrangement of the emitter pads 1332 of the vertical transistor 130 that are provided to facilitate the selection of the bioparticles by an electric field difference that is progressively distributed, but the present invention is not limited thereto.

In the vertical transistor 130 of the present embodiment, the emitter pads 1332 include a first pad 1332a arranged along an inner ring-shaped path P1, a centric pad 1332d arranged inside of the inner ring-shaped path P1, a second pad 1332b arranged along an outer ring-shaped path P2, and a third pad 1332c that is arranged along an additional ring-shaped path P3. The outer ring-shaped path P2 surrounds the inner ring-shaped path P1, and the additional ring-shaped path P3 is located between the inner ring-shaped path P1 and the outer ring-shaped path P2, but the present invention is not limited thereto. For example, in other embodiments of the present invention not shown in the drawings, the centric pad 1332d and/or the third pad 1332c can be omitted according to design requirements; or, a quantity of the additional ring-shaped path P3 between the inner ring-shaped path P1 and the outer ring-shaped path P2 can be at least two.

Specifically, a width of the centric pad 1332d, a width of the first pad 1332a, and a width of the second pad 1332b are different from each other, and a width of the third pad 1332c is within a range from the width of the first pad 1332a to the width of the second pad 1332b. In the present embodiment, widths of the emitter pads 1332 gradually decrease in a direction from the outer ring-shaped path P2 toward the centric pad 1332d. In other words, the emitter pads 1332 can be listed as follows in an order from largest to smallest in width: the second pad 1332b, the third pad 1332c, the first pad 1332a, and the centric pad 1332d, but the present invention is not limited thereto.

In addition, the specific distribution, quantity, and shape of the first pad 1332a, the second pad 1332b, and the third pad 1332c can be adjusted or changed according to design requirements, but the present invention is not limited thereto.

In summary, any one of the vertical transistors 130 of the pico-droplet generator 100 is configured to be irradiated by the light source mechanism 600 so as to allow a plurality of DEP forces to be applied to move the bioparticles (e.g., the at least one target bioparticle) through a distribution of the emitter pads 1332 and an electric field difference that is generated in the liquid specimen from non-uniform electric fields of the emitter pads 1332.

Accordingly, the photoelectric layer 13 of the bioparticle enrichment apparatus 1000 provided by the present embodiment has a specific structural design, so that the emitter pads 1332 of any one of the vertical phototransistors 130 can be used in a contactless photoelectric coupling manner to generate the electric fields jointly forming the electric field difference that is similar to a corona discharge, thereby enabling the emitter pads 1332 of any one of the vertical phototransistors 130 to accurately move (or capture) the at least one target bioparticle to any position.

### [Beneficial Effects of the Embodiments]

In conclusion, the configuration of the pico-droplet generator of the bioparticle enrichment apparatus provided by the present invention can be used to quickly and accurately implement a selection and enrichment process of the bioparticle (or the at least one target bioparticle) through the piezoelectric member. The pico-droplet generator in the present invention has an architecture that allows for mass-production of the same, thereby facilitating reduction of the overall cost of the bioparticle enrichment apparatus.

Moreover, the pico-droplet generator and the light source mechanism of the bioparticle enrichment apparatus provided by the present invention can be used in cooperation with each other to allow the pico-droplet emission region to quickly and accurately have the at least one target bioparticle therein, such that the at least one target bioparticle can be collected in the first container by forming the pico-droplet.

## Claims

1. A bioparticle enrichment apparatus (1000) provided for selecting at least one target bioparticle (B1) from a liquid specimen (L) having the bioparticles (B), the bioparticle enrichment apparatus (1000) comprising:
a pico-droplet generator (100) including:
a light sensing structure (1) including:
a first substrate (11);
a first electrode layer (12) and a photoelectric layer (13) respectively formed on two opposite sides of the first substrate (11); and
an insulating layer (14) covering the photoelectric layer (13);
a mating structure (2) spaced apart from the light sensing structure (1), wherein at least one of the mating structure (2) and the light sensing structure (1) is transparent, and the mating structure (2) includes a second substrate (21) and a second electrode layer (22) that is formed on the second substrate (21), and wherein the second electrode layer (22) faces toward the insulating layer (14);
a bonding layer (3) that is connected in-between the light sensing structure (1) and the mating structure (2) along a thickness direction (D1) so as to jointly define a selection channel (5) along a flowing direction (D2) perpendicular to the thickness direction (D1), wherein at least one of the mating structure (2) and the bonding layer (3) has an inlet (31) that is located at an upstream of the selection channel (5) and an outlet (32) that is located at a downstream of the selection channel (5), and wherein the bonding layer (3) has a selection hole (33) that is in spatial communication with the selection channel (5) along a dripping direction (D3) perpendicular to the thickness direction (D1) and the flowing direction (D2); and
a piezoelectric member (4) disposed on the bonding layer (3), wherein the piezoelectric member (4) and the selection hole (33) are respectively located at two opposite sides of the bonding layer (3) along the dripping direction (D3), and wherein the pico-droplet generator (100) has a pico-droplet emission region (R) defined as extending from the selection hole (33) toward the piezoelectric member (4);
a power device (200) electrically coupled to the first electrode layer (12) and the second electrode layer (22);
a pressure balance device (300) including a first valve (301) that is assembled to the inlet (31) and a second valve (302) that is assembled to the outlet (32), wherein the pressure balance device (300) is configured to control a velocity and a pressure of the liquid specimen (L) in the selection channel (5) through the first valve (301) and the second valve (302);
a camera device (400) corresponding in position to a viewable segment (51) of the selection channel (5), wherein the pico-droplet emission region (R) is arranged in the viewable segment (51), and the camera device (400) is configured to take a real-time image of the liquid specimen (L) in the viewable segment (51);
a control device (500) electrically coupled to the piezoelectric member (4), wherein, when the real-time image obtained by the camera device (400) shows that at least one target bioparticle (B1) is located in the pico-droplet emission region (R), the control device (500) allows the piezoelectric member (4) to output a pico-droplet (L1) by driving the liquid specimen (L) in the pico-droplet emission region (R) to pass through the selection hole (33), and wherein the pico-droplet (L1) covers the at least one target bioparticle (B1); and
a light source mechanism (600) being in cooperation with the pico-droplet generator (100);
wherein the photoelectric layer (13) includes:
a collector layer (131) formed on the first substrate (11), wherein the collector layer (131) includes a plurality of collector regions (1312) spaced apart from each other, and an end of each of the collector regions (1312) away from the first electrode layer (12) has a first slot-like portion (1313);
a plurality of base regions (1321) respectively formed in the first slot-like portions (1313) of the collector regions (1312), wherein an end of each of the base regions (1321) away from the first electrode layer (12) has a plurality of second slot-like portions (1322) spaced apart from each other; and
a plurality of emitter regions (1331) respectively formed in the base regions (1321), wherein each of the emitter regions (1331) includes a plurality of emitter pads (1332) respectively formed in the second slot-like portions (1322) of a corresponding one of the base regions (1321), wherein each of the base regions (1321), a corresponding one of the collector regions (1312), and a corresponding one of the emitter regions (1331) are jointly formed as a vertical transistor (130), and wherein the insulating layer (14) covers the vertical transistors (130) and separates the vertical transistors (130) from each other, and an end of each of the emitter pads (1332) away from the first electrode layer (12) is exposed from the insulating layer (14);
wherein any one of the vertical transistors (130) is configured to be irradiated by the light source mechanism (600) so as to enable a plurality of DEP forces to be applied to move the at least one target bioparticle (B1) through a distribution of the emitter pads (1332) and an electric field difference that is generated from non-uniform electric fields of the emitter pads (1332).

2. The bioparticle enrichment apparatus (1000) according to claim 1, wherein, in each of the vertical transistors (130), the emitter pads (1332) include a first pad (1332a) arranged along an inner ring-shaped path (P1) and a second pad (1332b) that is arranged along an outer ring-shaped path (P2) surrounding the inner ring-shaped path (P1), and a width of the first pad (1332a) is different from a width of the second pad (1332b).

3. The bioparticle enrichment apparatus (1000) according to claim 2, wherein, in each of the vertical transistors (130), the emitter pads (1332) include a third pad (1332c) arranged along an additional ring-shaped path (P3) located between the inner ring-shaped path (P1) and the outer ring-shaped path (P2), and a width of the third pad (1332c) is within a range from the width of the first pad (1332a) and the width of the second pad (1332b).

4. The bioparticle enrichment apparatus (1000) according to claim 1, wherein the pico-droplet generator (100) has a hydrophobic surface (34) surrounding the selection hole (33), and the pressure balance device (300) enables the liquid specimen (L) to form a liquid level (L2) in the selection hole (33) that is coplanar with an outer surface of the bonding layer (3).

5. The bioparticle enrichment apparatus (1000) according to claim 4, further comprising a first container (700) for receiving the pico-droplet (L1), wherein the pressure balance device (300) includes:
an air pump (303);
a switch (304) connected to the air pump (303);
a pressure balance bottle (305) being in fluid communication with the air pump (303) and the switch (304);
a liquid injection bottle (306) being in fluid communication with the switch (304) and the first valve (301), wherein the liquid injection bottle (306) enables the liquid specimen (L) received therein to be injected into the selection channel (5) through the first valve (301) and the inlet (31); and
a second container (307) being in fluid communication with the second valve (302).

6. The bioparticle enrichment apparatus (1000) according to claim 1, wherein a width (W33) of the selection hole (33) along the flowing direction (D2) is within a range from 40 µm to 300 µm, and a boundary of the viewable segment (51) is spaced apart from a center of the selection hole (33) along the flowing direction (D2) by a distance within a range from 100 µm to 300 µm.

7. The bioparticle enrichment apparatus (1000) according to claim 1, wherein the pico-droplet emission region (R) is arranged in a projection area defined by orthogonally projecting the selection hole (33) onto the piezoelectric member (4) along the dripping direction (D3).

8. The bioparticle enrichment apparatus (1000) according to claim 7, wherein a boundary of the pico-droplet emission region (R) is spaced apart from a center of the selection hole (33) along the dripping direction (D3) by a distance within a range from 50 µm to 200 µm.

## Patentansprüche

1. Biopartikel-Anreicherungsvorrichtung (1000), die zum Auswählen von mindestens einem Ziel-Biopartikel (B1) aus einer flüssigen Probe (L) bereitgestellt ist, die die Biopartikel (B) aufweist, wobei die Biopartikel-Anreicherungsvorrichtung (1000) aufweist:
einen Pikotropfengenerator (100), aufweisend:
eine Lichtsensorstruktur (1), aufweisend:
ein erstes Substrat (11),
eine erste Elektrodenschicht (12) und eine photoelektrische Schicht (13), die jeweils auf zwei entgegengesetzten Seiten des ersten Substrats (11) ausgebildet sind, und
eine Isolierschicht (14), die die photoelektrische Schicht (13) bedeckt,
eine Gegenstückstruktur (2), die von der Lichtsensorstruktur (1) im Abstand angeordnet ist, wobei mindestens eine von der Gegenstückstruktur (2) und der Lichtsensorstruktur (1) transparent ist und die Gegenstückstruktur (2) ein zweites Substrat (21) und eine zweite Elektrodenschicht (22) aufweist, die auf dem zweiten Substrat (21) ausgebildet ist, und wobei die zweite Elektrodenschicht (22) in Richtung zu der Isolierschicht (14) gewandt ist,
eine Verbindungsschicht (3), die zwischen der Lichtsensorstruktur (1) und der Gegenstückstruktur (2) entlang einer Dickenrichtung (D1) verbunden ist, um gemeinsam einen Auswahlkanal (5) entlang einer Strömungsrichtung (D2) senkrecht zur Dickenrichtung (D1) zu definieren, wobei mindestens eine von der Gegenstückstruktur (2) und der Verbindungsschicht (3) aufweist: einen Einlass (31), der sich stromaufwärts des Auswahlkanals (5) befindet, und einen Auslass (32), der sich stromabwärts des Auswahlkanals (5) befindet, und wobei die Verbindungsschicht (3) ein Auswahlloch (33) aufweist, das entlang einer Tropfrichtung (D3) senkrecht zur Dickenrichtung (D1) und zur Strömungsrichtung (D2) in räumlicher Kommunikation mit dem Auswahlkanal (5) ist, und
ein piezoelektrisches Element (4), das auf der Verbindungsschicht (3) angeordnet ist, wobei sich das piezoelektrische Element (4) und das Auswahlloch (33) jeweils an zwei entgegengesetzten Seiten der Verbindungsschicht (3) entlang der Tropfrichtung (D3) befinden, und wobei der Pikotropfengenerator (100) einen Pikotropfenemissionsbereich (R) aufweist, der definiert ist, um sich von dem Auswahlloch (33) in Richtung zu dem piezoelektrischen Element (4) zu erstrecken,
eine Leistungsvorrichtung (200), die elektrisch mit der ersten Elektrodenschicht (12) und der zweiten Elektrodenschicht (22) gekoppelt ist,
eine Druckausgleichsvorrichtung (300) aufweisend ein erstes Ventil (301), das am Einlass (31) montiert ist, und ein zweites Ventil (302), das am Auslass (32) montiert ist, wobei die Druckausgleichsvorrichtung (300) konfiguriert ist, um eine Geschwindigkeit und einen Druck der flüssigen Probe (L) im Auswahlkanal (5) durch das erste Ventil (301) und das zweite Ventil (302) zu steuern,
eine Kameravorrichtung (400), die in ihrer Position mit einem sichtbaren Segment (51) des Auswahlkanals (5) korrespondiert, wobei der Pikotropfenemissionsbereich (R) in dem sichtbaren Segment (51) angeordnet ist und die Kameravorrichtung (400) konfiguriert ist, um ein Echtzeitbild der flüssigen Probe (L) in dem sichtbaren Segment (51) aufzunehmen,
eine Steuervorrichtung (500), die elektrisch mit dem piezoelektrischen Element (4) gekoppelt ist, wobei, wenn das von der Kameravorrichtung (400) erhaltene Echtzeitbild zeigt, dass sich mindestens ein Ziel-Biopartikel (B1) im Pikotropfenemissionsbereich (R) befindet, die Steuervorrichtung (500) es dem piezoelektrischen Element (4) ermöglicht, einen Pikotropfen (L1) auszugeben, durch Ansteuern der flüssigen Probe (L) in dem Pikotropfenemissionsbereich (R), um durch das Auswahlloch (33) hindurchzutreten, und wobei der Pikotropfen (L1) das mindestens eine Ziel-Biopartikel (B1) bedeckt, und
einen Lichtquellenmechanismus (600), der mit dem Pikotropfengenerator (100) zusammenwirkt,
wobei die photoelektrische Schicht (13) aufweist:
eine auf dem ersten Substrat (11) ausgebildete Kollektorschicht (131), wobei die Kollektorschicht (131) eine Mehrzahl von voneinander im Abstand angeordneten Kollektorbereichen (1312) aufweist und ein von der ersten Elektrodenschicht (12) abgewandtes Ende jedes der Kollektorbereiche (1312) einen ersten schlitzartigen Abschnitt (1313) aufweist,
eine Mehrzahl von Basisbereichen (1321), die jeweils in den ersten schlitzartigen Abschnitten (1313) der Kollektorbereiche (1312) ausgebildet sind, wobei ein von der ersten Elektrodenschicht (12) abgewandtes Ende von jedem der Basisbereiche (1321) eine Mehrzahl von voneinander im Abstand angeordneten zweiten schlitzartigen Abschnitten (1322) aufweist, und
eine Mehrzahl von Emitterbereichen (1331), die jeweils in den Basisbereichen (1321) ausgebildet sind, wobei jeder der Emitterbereiche (1331) eine Mehrzahl von Emitterpads (1332) aufweist, die jeweils in den zweiten schlitzartigen Abschnitten (1322) eines korrespondierenden der Basisbereiche (1321) ausgebildet sind, wobei jeder der Basisbereiche (1321), ein korrespondierender der Kollektorbereiche (1312) und ein korrespondierender der Emitterbereiche (1331) gemeinsam als vertikaler Transistor (130) ausgebildet sind, und wobei die Isolierschicht (14) die vertikalen Transistoren (130) bedeckt und die vertikalen Transistoren (130) voneinander trennt, und ein von der ersten Elektrodenschicht (12) abgewandtes Ende von jedem der Emitterpads (1332) aus der Isolierschicht (14) freigelegt ist,
wobei irgendeiner der vertikalen Transistoren (130) konfiguriert ist, um von dem Lichtquellenmechanismus (600) bestrahlt zu werden, um die Ausübung einer Mehrzahl von DEP-Kräften zu ermöglichen, um das mindestens eine Ziel-Biopartikel (B1) durch eine Verteilung der Emitterpads (1332) und einer elektrischen Felddifferenz, die aus ungleichmäßigen elektrischen Feldern der Emitterpads (1332) erzeugt wird, zu bewegen.

2. Biopartikel-Anreicherungsvorrichtung (1000) gemäß Anspruch 1, wobei in jedem der vertikalen Transistoren (130) die Emitterpads (1332) aufweisen: ein erstes Pad (1332a), das entlang eines inneren ringförmigen Pfades (P1) angeordnet ist, und ein zweites Pad (1332b), das entlang eines äußeren ringförmigen Pfades (P2) angeordnet ist, der den inneren ringförmigen Pfad (P1) umgibt, und eine Breite des ersten Pads (1332a) sich von einer Breite des zweiten Pads (1332b) unterscheidet.

3. Biopartikel-Anreicherungsvorrichtung (1000) gemäß Anspruch 2, wobei in jedem der vertikalen Transistoren (130) die Emitterpads (1332) ein drittes Pad (1332c) aufweisen, das entlang eines zusätzlichen ringförmigen Pfades (P3) angeordnet ist, der sich zwischen dem inneren ringförmigen Pfad (P1) und dem äußeren ringförmigen Pfad (P2) befindet, und wobei eine Breite des dritten Pads (1332c) innerhalb eines Bereichs von der Breite des ersten Pads (1332a) und der Breite des zweiten Pads (1332b) ist.

4. Biopartikel-Anreicherungsvorrichtung (1000) gemäß Anspruch 1, wobei der Pikotropfengenerator (100) eine das Auswahlloch (33) umgebende hydrophobe Fläche (34) hat, und die Druckausgleichsvorrichtung (300) es ermöglicht, dass die flüssige Probe (L) in dem Auswahlloch (33) einen Flüssigkeitsspiegel (L2) bildet, der koplanar mit einer Außenfläche der Verbindungsschicht (3) ist.

5. Biopartikel-Anreicherungsvorrichtung (1000) gemäß Anspruch 4, ferner aufweisend einen ersten Behälter (700) zum Aufnehmen des Pikotropfens (L1), wobei die Druckausgleichsvorrichtung (300) aufweist:
eine Luftpumpe (303),
einen Schalter (304), der mit der Luftpumpe (303) verbunden ist,
eine Druckausgleichsflasche (305), die in Fluidkommunikation mit der Luftpumpe (303) und dem Schalter (304) ist,
eine Flüssigkeitseinspritzflasche (306), die in Fluidkommunikation mit dem Schalter (304) und dem ersten Ventil (301) ist, wobei die Flüssigkeitseinspritzflasche (306) es ermöglicht, die darin aufgenommene flüssige Probe (L) durch das erste Ventil (301) und den Einlass (31) in den Auswahlkanal (5) einzuspritzen, und
einen zweiten Behälter (307), der in Fluidverbindung mit dem zweiten Ventil (302) ist.

6. Biopartikel-Anreicherungsvorrichtung (1000) gemäß Anspruch 1, wobei eine Breite (W33) des Auswahllochs (33) entlang der Strömungsrichtung (D2) innerhalb eines Bereichs von 40 µm bis 300 µm ist, und eine Grenze des sichtbaren Segments (51) entlang der Strömungsrichtung (D2) um einen Abstand innerhalb eines Bereichs von 100 µm bis 300 µm von einer Mitte des Auswahllochs (33) beabstandet ist.

7. Biopartikel-Anreicherungsvorrichtung (1000) gemäß Anspruch 1, wobei der Pikotropfenemissionsbereich (R) in einem Projektionsbereich angeordnet ist, der durch orthogonale Projektion des Auswahllochs (33) auf das piezoelektrische Element (4) entlang der Tropfrichtung (D3) definiert ist.

8. Biopartikel-Anreicherungsvorrichtung (1000) gemäß Anspruch 7, wobei eine Grenze des Pikotropfenemissionsbereichs (R) von einer Mitte des Auswahllochs (33) entlang der Tropfrichtung (D3) um einen Abstand innerhalb eines Bereichs von 50 µm bis 200 µm beabstandet ist.

## Revendications

1. Appareil d'enrichissement en bioparticules (1000) destiné à sélectionner au moins une bioparticule cible (B1) à partir d'un échantillon liquide (L) contenant les bioparticules (B), l'appareil d'enrichissement en bioparticules (1000) comprenant :
un générateur de pico-gouttelettes (100) comprenant :
une structure de détection de lumière (1) comprenant :
un premier substrat (11) ;
une première couche d'électrode (12) et une couche photoélectrique (13) formées respectivement sur deux côtés opposés du premier substrat (11) ; et
une couche isolante (14) recouvrant la couche photoélectrique (13) ;
une structure complémentaire (2) espacée de la structure de détection de lumière (1), dans lequel au moins l'une parmi la structure complémentaire (2) et la structure de détection de lumière (1) est transparente, et la structure complémentaire (2) comprend un deuxième substrat (21) et une deuxième couche d'électrode (22) formée sur le deuxième substrat (21), et dans lequel la deuxième couche d'électrode (22) est tournée vers la couche isolante (14) ;
une couche de liaison (3) qui est connectée entre la structure de détection de lumière (1) et la structure complémentaire (2) le long d'une direction d'épaisseur (D1) de manière à définir conjointement un canal de sélection (5) le long d'une direction d'écoulement (D2) perpendiculaire à la direction d'épaisseur (D1), dans lequel au moins l'une parmi la structure complémentaire (2) et la couche de liaison (3) comporte une entrée (31) située en amont du canal de sélection (5) et une sortie (32) située en aval du canal de sélection (5), et dans lequel la couche de liaison (3) comporte un trou de sélection (33) qui est en communication spatiale avec le canal de sélection (5) le long d'une direction d'égouttement (D3) perpendiculaire à la direction d'épaisseur (D1) et à la direction d'écoulement (D2) ; et
un élément piézoélectrique (4) disposé sur la couche de liaison (3), dans lequel l'élément piézoélectrique (4) et le trou de sélection (33) sont respectivement situés sur deux côtés opposés de la couche de liaison (3) le long de la direction d'égouttement (D3), et dans lequel le générateur de pico-gouttelettes (100) comporte une région d'émission de pico-gouttelettes (R) définie comme s'étendant depuis le trou de sélection (33) vers l'élément piézoélectrique (4) ;
un dispositif de puissance (200) couplé électriquement à la première couche d'électrode (12) et à la deuxième couche d'électrode (22) ;
un dispositif d'équilibrage de pression (300) comprenant une première vanne (301) qui est montée sur l'entrée (31) et une deuxième vanne (302) qui est montée sur la sortie (32), dans lequel le dispositif d'équilibrage de pression (300) est configuré pour contrôler une vitesse et une pression de l'échantillon liquide (L) dans le canal de sélection (5) par l'intermédiaire de la première vanne (301) et de la deuxième vanne (302) ;
un dispositif de caméra (400) correspondant en position à un segment visible (51) du canal de sélection (5), dans lequel la région d'émission de pico-gouttelettes (R) est disposée dans le segment visible (51), et le dispositif de caméra (400) est configuré pour prendre une image en temps réel de l'échantillon liquide (L) dans le segment visible (51) ;
un dispositif de commande (500) couplé électriquement à l'élément piézoélectrique (4), dans lequel, lorsque l'image en temps réel obtenue par le dispositif de caméra (400) montre qu'au moins une bioparticule cible (B1) est située dans la région d'émission de pico-gouttelettes (R), le dispositif de commande (500) permet à l'élément piézoélectrique (4) d'émettre une pico-gouttelette (L1) en entraînant l'échantillon liquide (L) dans la région d'émission de pico-gouttelettes (R) à passer à travers le trou de sélection (33), et dans lequel la pico-gouttelette (L1) recouvre ladite au moins une bioparticule cible (B1) ; et
un mécanisme de source lumineuse (600) coopérant avec le générateur de pico-gouttelettes (100) ;
dans lequel la couche photoélectrique (13) comprend :
une couche collectrice (131) formée sur le premier substrat (11), dans lequel la couche collectrice (131) comprend une pluralité de régions collectrices (1312) espacées les unes des autres, et une extrémité de chacune des régions collectrices (1312) éloignée de la première couche d'électrode (12) présente une première partie de type de fente (1313) ;
une pluralité de régions de base (1321) formées respectivement dans les premières parties de type de fente (1313) des régions collectrices (1312), dans lequel une extrémité de chacune des régions de base (1321) éloignée de la première couche d'électrode (12) comporte une pluralité de deuxièmes parties de type de fente (1322) espacées les unes des autres ; et
une pluralité de régions d'émetteur (1331) formées respectivement dans les régions de base (1321), dans lequel chacune des régions d'émetteur (1331) comprend une pluralité de plots d'émetteur (1332) formés respectivement dans les deuxièmes parties de type de fente (1322) d'une correspondante des régions de base (1321), dans lequel chacune des régions de base (1321), une correspondante des régions collectrices (1312) et une correspondante des régions d'émetteur (1331) sont formées conjointement en tant que transistor vertical (130), et dans lequel la couche isolante (14) recouvre les transistors verticaux (130) et sépare les transistors verticaux (130) les uns des autres, et une extrémité de chacun des plots d'émetteur (1332) éloignée de la première couche d'électrode (12) est exposée à partir de la couche isolante (14) ;
dans lequel l'un quelconque des transistors verticaux (130) est configuré pour être irradié par le mécanisme de source lumineuse (600) de manière à permettre l'application d'une pluralité de forces DEP pour déplacer ladite au moins une bioparticule cible (B1) à travers une distribution des plots d'émetteur (1332) et une différence de champ électrique générée par les champs électriques non uniformes des plots d'émetteur (1332).

2. Appareil d'enrichissement en bioparticules (1000) selon la revendication 1, dans lequel, dans chacun des transistors verticaux (130), les plots d'émetteur (1332) comprennent un premier plot (1332a) disposé le long d'un trajet annulaire intérieur (P1) et un deuxième plot (1332b) disposé le long d'un trajet annulaire extérieur (P2) entourant le trajet annulaire intérieur (P1), et une largeur du premier plot (1332a) est différente d'une largeur du deuxième plot (1332b).

3. Appareil d'enrichissement en bioparticules (1000) selon la revendication 2, dans lequel, dans chacun des transistors verticaux (130), les plots d'émetteur (1332) comprennent un troisième plot (1332c) disposé le long d'un trajet annulaire supplémentaire (P3) situé entre le trajet annulaire intérieur (P1) et le trajet annulaire extérieur (P2), et une largeur du troisième plot (1332c) se situe dans une plage de la largeur du premier plot (1332a) et la largeur du deuxième plot (1332b).

4. Appareil d'enrichissement en bioparticules (1000) selon la revendication 1, dans lequel le générateur de pico-gouttelettes (100) comporte une surface hydrophobe (34) entourant le trou de sélection (33), et le dispositif d'équilibrage de pression (300) permet à l'échantillon liquide (L) de former un niveau de liquide (L2) dans le trou de sélection (33) qui est coplanaire avec une surface extérieure de la couche de liaison (3).

5. Appareil d'enrichissement en bioparticules (1000) selon la revendication 4, comprenant en outre un premier récipient (700) destiné à recevoir la pico-gouttelette (L1), dans lequel le dispositif d'équilibrage de pression (300) comprend :
une pompe à air (303) ;
un commutateur (304) relié à la pompe à air (303) ;
une bouteille d'équilibrage de pression (305) en communication fluidique avec la pompe à air (303) et le commutateur (304) ;
une bouteille d'injection de liquide (306) en communication fluidique avec le commutateur (304) et la première vanne (301), dans lequel la bouteille d'injection de liquide (306) permet à l'échantillon liquide (L) reçu à son intérieur d'être injecté dans le canal de sélection (5) par l'intermédiaire de la première vanne (301) et de l'entrée (31) ; et
un deuxième récipient (307) en communication fluidique avec la deuxième vanne (302).

6. Appareil d'enrichissement en bioparticules (1000) selon la revendication 1, dans lequel une largeur (W33) du trou de sélection (33) le long de la direction d'écoulement (D2) se situe dans une plage allant de 40 µm à 300 µm, et une limite du segment visible (51) est espacée d'un centre du trou de sélection (33) le long de la direction d'écoulement (D2) d'une distance comprise dans une plage allant de 100 µm à 300 µm.

7. Appareil d'enrichissement en bioparticules (1000) selon la revendication 1, dans lequel la région d'émission de pico-gouttelettes (R) est disposée dans une zone de projection définie par la projection orthogonale du trou de sélection (33) sur l'élément piézoélectrique (4) le long de la direction d'égouttement (D3).

8. Appareil d'enrichissement en bioparticules (1000) selon la revendication 7, dans lequel une limite de la région d'émission de pico-gouttelettes (R) est espacée d'un centre du trou de sélection (33) le long de la direction d'égouttement (D3) d'une distance comprise dans une plage allant de 50 µm à 200 µm.
